Europäisches Patentamt

European Patent Office

Office européen des brevets

(19)

(11) Publication number : **0 115 911 B2**

# NEW EUROPEAN PATENT SPECIFICATION

(12)

(45) Date of publication of the new patent specification : **27.10.93 Bulletin 93/43**

(51) Int. Cl.⁵ : **A61M 1/28, C12P 19/14**

(21) Application number : **84300134.8**

(22) Date of filing : **10.01.84**

(54) Peritoneal dialysis solutions and preparation thereof.

(30) Priority : **12.01.83 GB 8300718**

(43) Date of publication of application :
**15.08.84 Bulletin 84/33**

(45) Publication of the grant of the patent :
**23.11.88 Bulletin 88/47**

(45) Mention of the opposition decision :
**27.10.93 Bulletin 93/43**

(84) Designated Contracting States :
**AT BE CH DE FR IT LI LU NL SE**

(56) References cited :
**EP-A- 0 076 355**
**WO-A-82/03329**
**WO-A-83/00087**
**GB-A- 2 042 547**
**US-A- 3 783 100**
**US-A- 3 912 590**
**US-A- 4 239 041**
**US-A- 4 308 255**
**US-A- 4 339 433**

(56) References cited :
"Frontiers in Peritoneal Dialysis", Winchester et al., Feb. 1986, pp 231 to 240
"Ultrafiltration with an isosmotic solution during long peritoneal dialysis exchanges", The Lancet, Mistry et al., July 1987, pp 178 to 182
"Metabolism of Parenteral Glucose Oligosaccharides in Man", Young et al, Journal of Parenteral and Enteral Nutrition, Vol. 5, No. 5, 1981, pp 396 to 377
"Sustained Ultrafiltration with Polymer Dialysate during Long Dwell Peritoneal Dialysis Exchanges", Twardowski et al., Kidney International, 21, 1982 (4), p 181

(73) Proprietor : **M.L. LABORATORIES PLC**
**Marcol House, 293 Regent Street**
**London W1R 7PD (GB)**

(72) Inventor : **Milner, Jeremiah**
**22 Albert Road**
**Cheltenham Gloucestershire (GB)**

(74) Representative : **Harrison, Michael Robert**
**DIBB LUPTON BROOMHEAD and PRIOR 117**
**The Headrow**
**Leeds LS1 5JX (GB)**

EP 0 115 911 B2

## Description

This invention relates to peritoneal dialysis, which is now a well-established method for the treatment of patients suffering from acute or chronic renal failure or insufficiency.

Peritoneal dialysis is an alternative to haemodialysis. In haemodialysis, the patient's blood is treated outside the patient's body to effect removal of water and waste products, such as urea and creatinine, by subjecting the blood to a process of dialysis in an artificial kidney machine. In peritoneal dialysis the patient's blood is not withdrawn from the body; instead, a dialysing solution is introduced into the abdominal cavity and removal of water and waste products is effected by dialysis across the peritoneal membrane.

In order to create the osmotic pressure which is necessary to cause peritoneal dialysis, the dialysing solution must contain an osmotic agent, which has in the past normally been dextrose.

It has been recognised for some time that the use of dextrose as the osmotic agent is not entirely satisfactory, mainly because the dextrose can pass from the abdominal cavity through the peritoneal membrane, causing an undesirably rapid drop in the osmotic pressure, so that the dialysing solution has to be removed and replaced by fresh solution more frequently than is desirable and also causing an increase in the level of dextrose in the blood, which is often injurious to the patient.

It has previously been suggested that the performance of peritoneal dialysis solutions might be improved if dextrose were replaced, as the osmotic agent, by a mixture of glucose polymers. The first such suggestion appears to have been at a seminar on renal disease at Manchester Royal Infirmary on July 1st, 1981. Subsequently, WO-A-8300087 (published 20.01.1983) disclosed a peritoneal dialysis solution comprising a water solution of physiologically tolerable pH, having physiological salts and metabolizable carbohydrate polymers having an average degree of polymerisation (D.P.) of at least 4 in concentrations sufficient safely to effect the removal of solutes and water from a patient by peritoneal dialysis. The polymers may be glucose polymers having an average D.P. of 4 to 10.

The present invention provides the use in the preparation of a peritoneal dialysis composition of an osmotic agent comprising a glucose polymer mixture, said mixture being derived from the hydrolysis of starch and including at least 20% by weight of glucose polymers of D.P. greater than 12.

Glucose polymer mixtures containing at least 15% by weight of glucose polymers of D.P. greater than 12 are not new per se. Many disclosures of such mixtures exist in the literature dealing with starch hydrolysis and products derived therefrom; an example of such a dislosure is specification WO-A-8203329. However, it has not previously been proposed to use such a mixture as an osmotic agent in a peritoneal dialysis solution.

Prior to the present invention it had been thought that glucose polymer mixtures for use in peritoneal dialysis solutions should preferably have little or no content of glucose polymers of relatively high D.P. There were several reasons for this. The concentration of an osmotic agent in a solution which is required to produce a given osmotic pressure is directly related to its molecular weight and it would therefore be expected that with an osmotic agent consisting of a glucose polymer mixture containing a substantial amount of high molecular weight polymers the concentration of the osmotic agent required to produce the desired osmotic pressure would be so high as to make the solution too viscous for it to be of practical value in peritoneal dialysis. This has now been found not to be the case. Another disadvantage that was expected to result from the presence in the osmotic agent of high molecular weight glucose polymers was that passage into the blood of the patient of such polymers would be injurious, at least in the treatment of chronic renal failure, because of an expected inability of the patent to metabolise these polymers as efficiently as glucose polymers of lower molecular weight. This expectation has also proved to be unfounded.

Moreover, it has been found in clinical tests that the presence in the osmotic agent of glucose polymers of D.P. greater than 12 is advantageous. Firstly, these polymers do not readily pass through the peritoneal membrane during dialysis. As a result, the osmolarity of the dialysing solution drops less rapidly than would be expected so that the solution can be allowed to remain in the peritoneum for a longer time than would otherwise be feasible. Secondly, the glucose polymers of D.P. greater than 12 are apparently more effective as osmotic agents than would be indicated by calculations based on the standard assumption that each molecule of such a polymer would be osmotically the equivalent of one molecule of dextrose or any other compound. This is demonstrated by the fact that the dialysing solutions of the present invention can be satisfactorily used in peritoneal dialysis with a concentration of glucose polymers in the solutions considerably lower than would have been expected. The dextrose solutions used in the past have commonly contained about 4 or 5% of dextrose. The osmolarity of a solution is normally calculated by reference to the number of molecules in the solution; thus, it would be expected that if the dextrose were replaced by maltose the concentration of maltose (which has a molecular weight almost twice that of dextrose) would need to be about double that of the dextrose, i.e. 8 or 10%. When glucose polymer mixtures of average molecular weight higher than that of maltose were used as the osmotic agent, even higher concentrations would be expected to be necessary. However, it has been found that in practice the dialysing solutions of this invention can create a sufficient osmotic pressure for peritoneal dialysis without substantially increasing the concentration of the glucose polymer mixture above that of dextrose in the solutions previously used. The reason for this is not yet known.

It is well known that glucose polymer mixtures can be prepared by hydrolysis of starch, as disclosed, for example, in GB-A-1,444,901 and GB-A-2,008,406.

In these known processes, one or more of the process steps is carried out in order to remove carbohydrate material that has too low or too high a molecular weight. Further, the known processes usually comprise a very large number of steps and/or comprise a change in the state (liquid or solid) of the material being treated which make it difficult or impossible to carry out the process continuously.

We have now found that by more careful control of the enzymatic hydrolyisis of the starch, more particularly by carrying out this hydrolysis in two stages in a particular way, little or no undesirable carbohydrate material is produced so that it is not necessary to separate hydrolysis products having too low or too high a molecular weight. The overall process is, consequently, much simplified and is well suited for continuous and substantially automated operation.

According to one aspect of the present invention, there is provided a process for the preparation of a sterile aqueous solution of a glucose polymer mixture, the mixture containing less than 5% by weight of glucose and more than 15% by weight of glucose polymers having a degree of polymerisation of more than 12, which comprises

(i) gelatinising clean, pre-washed starch containing not more than 5% of 1,6-linkages in aqueous suspension which from 0.05 to 0.13%, based on the weight of the suspension, of at least one thermophilic amylase at a pH of from 6.0 to 6.8 and a temperature of 95° to 100°C,

(ii) adjusting the pH of the aqueous mixture from step (i) to 4.5 to 5.0 by the addition of a physiologically acceptable organic acid and increasing the concentration of thermophilic amylase to from 0.15 to 0.25%, on the same basis, by the addition of further amylase and effecting dextrinisation of the gelatinised starch at a temperature of 90° to 95°C so as to obtain a hydrolysis product predominantly consisting of glucose polymers having a degree of polymerisation of more than 12 and containing less than 1% by weight of glucose and less than 2% by weight of each of maltose and maltotriose,

(iii) adjusting the pH of the aqueous mixture from step (ii), if required, to 3.5 to 4.5 by the addition of a physiologically acceptable organic acid and effecting hydrolysis of the dextrinised starch at a temperature of 65° to 75°C by means of a bacterial alpha-amylase which is capable, under these conditions, of producing less than 5% of glucose, based on the weight of the dextrinised starch, and continuing hydrolysis under these conditions until a glucose polymer mixture of the desired composition is obtained, and

(iv) adjusting the pH of the aqueous mixture from step (iii) to 2.0 to 3.0 by the addition of a physiologically acceptable organic acid and heating the mixture at a temperature of 96° to 100°C so as to inactivate the enzymes.

We have further found that by appropriate choice of the amount of bacterial alpha-amylase used in step (iii), glucose polymer mixtures having a varying distribution of polymer sizes can be obtained, as will be illustrated in the Examples given below.

These varying polymer size distributions can be obtained by using concentrations of the bacterial alpha-amylase of from about 0.1 to about 3.0%, based on the total weight of the aqueous mixture.

The process according to the invention is preferably carried out so as to produce a relatively concentrated solution of the glucose polymer mixture, that is containing 65 to 80% w/v, of the polymer mixture; such solutions having a specific gravity of from 1.275 to 1.300. With the addition of a conventional amount of a physiologically acceptable preservative, for example 0.1% by weight of sodium benzoate, and packaged in a sealed container under sterile conditions, such solutions are storage stable at temperatures of from 0 to 100°C for up to 2 years. When the containers are opened, the solutions are usable, when kept at room temperature, for up to 2 months.

The solutions just described, that is containing 65 to 80% w/v of a glucose polymer mixture having one of the preferred polymer mixture compositions described, are novel and constitute a further aspect of the present invention.

The process according to the invention will now be described in greater detail.

The starch used as the starting material of the process should, as stated, be a clean, pre-washed starch containing not more than 5% of 1,6-linkages. The starch may be derived from corn, rice or barley, corn starch being in general the most readily available. In the United States so-called "green" starch is commercially available, this being an aqueous suspension of washed corn starch, and this is also a suitable starting material.

The washed starch should be low in sodium, that is contain less than 5 milliequivalents of sodium per 100 g, and low in proteins, that is contain less than 0.1% of protein. The starch should be assayed for insecticides, weedkillers, and plant hormones and should be free of these products.

Clean, pre-washed starch meeting the requirements specified is commercially available from a number of suppliers.

The initial aqueous suspension preferably contains from 50 to 60% by weight of starch; with evaporative losses of water during the various stages of the process, this range of initial solids concentrations gives the above-mentioned preferred solids contents, i.e. 65-80% by weight of the final solution.

Step (i) of the process is preferably carried out with approximately 0.1% of the thermophilic amylase at a pH of approximately 6.0 and a temperature of approximately 98°C. A number of thermophilic amylases are commercially available and any of them can, in principle, be used in steps (i) and (ii). Suitable amylases include, for example, those available under the trade names "Termamyl" and "Fungamyl" from Novo, Denmark; of these "Termamyl" is particularly preferred.

Step (i) of the process, the gelatinisation of the starch, is completed quite rapidly, for example in 5-10 minutes.

To effect step (ii), the dextrinisation of the gelatinised starch, the concentration of the amylase is substantially increased, preferably doubled, to within the range 0.15 to 0.25%, and a slightly lower temperature, preferably approximately 95°C, and a lower pH, preferably about 4.5, is used. The organic acid used to effect the reduction in the pH (and also for steps (iii) and (iv)) may, for example, be citric acid, lactic acid or acetic acid, of which the first is preferred.

Step (ii) takes considerably longer than step (i); the former typically takes from 2-4 hours.

The mixture is then passed on to step (iii). The pH is reduced, if required, to within the range 3.4-4.5, preferably to approximately 4.5, and a lower temperature, that is 65°-75°C, preferably approximately 70°C is used. At these temperatures, thermophilic amylases are substantially inactive and a bacterial alpha-amylase which is active at these temperatures and which is capable of producing less than 5% of glucose, based on the weight of the dextrinised starch, is used. A particularly preferred bacterial alpha-amylase for this purpose is that available under the trade name "Ban" from Novo, Denmark, but other suitable bacterial alpha-amylases can be used, if desired. Step (iii) typically takes about 4 hours.

In step (iv), the pH is again reduced, preferably to a pH of approximately 2.5, and the aqueous mixture is heated at a temperature of 96° to 100°C, preferably approximately 98°C, to effect complete deactivation of the enzymes. This step typically takes about 3 hours.

It is preferred, but not essential, to filter the aqueous mixture between steps (ii) and (iii). A fine pore glass filter is preferred, for example having a porosity of 1H.

It will be appreciated that since there is no removal of unwanted products in the process according to the invention, it can readily be carried out continuously using a number of reactors or tanks connected in series, each step being carried out in a separate reactor and with means for metering the required amounts of enzyme and acid to the various steps and means for thermostatically controlling the temperature in the reactors. Such a process is well suited to automated control.

The final product may be discharged directly from the final reactor or tank to a sterile filling station where it is filled into suitable containers, such as plastics sachets, which are then sealed.

The amylases used in the process according to the invention sometimes impart an undesirable odour or flavour and/or discoloration to the product and for this reason it is desirable to use the minimum amount of amylase (within the ranges specified above). If despite this precaution, the product is subject to an undesirable odour or flavour and/or discoloration, it can readily be de-odorised and/or de-colourised by contact with a suitable inert absorbent or adsorbent, such as activated carbon.

It is also preferred to add a small amount of a permitted preservative, for example 0.1% by weight of sodium benzoate prior to packing.

The solutions of glucose polymer mixtures obtained by the process according to the invention are completely non-antigenic.

The glucose polymer solution is preferably adjusted to a pH of 7.0, in this case preferably with sodium acetate, and electrolytes are added thereto, prior to sterile packing. The concentrated solution can be diluted when required to give a peritoneal dialysis solution having an appropriate concentration of glucose polymers. The dilution can be effected by means of an aqueous diluent containing any solutes known to be desirable in peritoneal dialysis solutions, such as lactates, acetates and amino acids, the particular composition of the diluent being at the discretion of the clinician and not forming part of the present invention.

Alternatively, the concentrated solution of the glucose polymer mixture may be spray-dried to produce a powdered form of the mixture which can, when desired, be dissolved in a suitable aqueous solution to form a peritoneal dialysis solution.

The concentration of the glucose polymer mixture in the peritoneal dialysis solutions of the invention is varied in accordance with the nature of the required therapy and may, for example, range from about 2% to about 15%. For use in continuous ambulatory peritoneal dialysis a concentration of from 2 to 5% is generally appropriate, with a dwell time in the abdominal cavity of from 5 to 8 hours. For treatment of acute renal failure a concentration of from 5 to 10%, with dwell times of up to 6 hours, is indicated. In a situation where rapid withdrawal of water is of paramount importance concentrations of 10 to 15% may be desirable. These figures are given only as a general guide because ultimately the choice of the composition of the peritoneal dialysis solution most suited to the needs of a particular patient must rest with the clinician.

Although it is not yet clear why the use of an osmotic agent comprising a glucose polymer mixture including a relatively high proportion of high molecular weight material is advantageous it seems likely that this is due to a phenomenon, which may be described as colloidal osmosis, caused by the apparent ability of the high molecular weight material to continue attracting water and its solutes across the peritoneal membrane for a longer period than lower molecular weight material would do. Presumably, this is partially because of the relative inability of the high molecular weight polymers to diffuse through the peritoneal membrane but it may also derive from hydrogen bonding between water molecules and the higher molecular weight polymers which in effect serves to be equivalent to removal of water from the dialysate in the abdominal cavity. Such hydrogen bonding would also tend to diminish the ability of the molecules to pass through the peritoneal membrane in that it would increase the size and the diameter of the molecules.

As stated above, the glucose polymer mixture used as the osmotic agent includes at least 20% by weight of glucose polymers of D.P. greater than 12. Preferably it includes from 20 to 50% of glucose polymers of D.P. greater than 12. Most such glucose polymer mixtures can be produced by the process of the invention but, in cases where this is not so, the desired distribution of polymers can be achieved by fractionation techniques well known in the art of producing glucose polymer mixtures.

The process of the invention produces glucose polymer mixtures containing less than 5% of glucose; such a concentration of glucose is desirable, especially as many patients needing peritoneal dialysis are diabetic. However, if the needs of a patient are such that a higher concentration of glucose is desirable it is a simple matter to add dextrose to the solutions of the invention to whatever extent in appropriate. For most purpose, however, it is desirable that the glucose polymer mixture should contain less than 5%, preferably less than 3% of glucose.

In order that the invention may be more fully understood, the following examples are given by way of illustration only:

Examples 1-3

A commercially available clean, pre-washed soluble corn starch was suspended in water with agitation to give a 60% by weight suspension.

Step (i), gelatinisation, was carried out in the presence of 0.1%, based on the weight of the suspension, of "Termamyl" thermophilic amylase at a pH of 6.0 and a temperature of 98°C for 5 minutes, in a first reactor.

The mixture from step (i) was passed to a second reactor and citric acid was metered into it to reduce the pH to 4.5 and additional "Termamyl" was added to raise its concentration and 0.2%, on the same basis. Step (ii), dextrinisation, was carried out at a temperature of 95°C for 4 hours.

The mixture from step (ii) was then passed to a third reactor in which step (iii), hydrolysis, was effected with "Ban" amylase at a pH 4.5.

Different amounts of the enzyme were used in each example and the conditions used and the polymer composition of the products obtained are summarised in Table 1 below.

TABLE 1

|  | Ex. 1. | Ex. 2. | Ex. 3. (comparative) |
| --- | --- | --- | --- |
| Initial solids, w/v | 70% | 70% | 70% |
| pH | 4.5 | 4.5 | 4.5 |
| Temp. | 70°C | 70°C | 70°C |
| "Ban" enzyme | 0.12% | 0.6% | 3.0% |
| Reaction time | 4 hrs. | 4 hrs. | 4 hrs. |

The mixtures from step (iii) were then passed to a fourth reactor and citric acid was metered into them to give a pH of 2.5. In the fourth reactor step (iv) was carried out by heating to 98°C for 3 hours.

The solids content of the final solutions was analysed and the results obtained are summarised in Table 2 below (G1=glucose, G2=polymer with 2 glucose units, G3=polymer with 3 glucose units, etc).

TABLE 2
Examples 1—3

|  | Ex. 1. | Ex. 2. | Ex. 3. (comparative) |
|---|---|---|---|
| G1 | 1.4 | 2.6 | 4.0 |
| G2 | 6.8 | 11.0 | 16.6 |
| G3 | 11.3 | 17.6 | 26.2 |
| G4 | 6.9 | 8.9 | 10.6 |
| G5 | 8.1 | 13.0 | 11.5 |
| G6 | 16.8 | 14.7 | 5.1 |
| G7 | 10.2 | 2.3 | 1.8 |
| G8 | 1.7 | 1.3 | 2.0 |
| G9 | 0.8 | 1.6 | 1.9 |
| G10 | 0.8 | 1.4 | 1.7 |
| G11 | 0.8 | 1.3 | 1.4 |
| G12 | 0.8 | 1.2 | 1.1 |
| G13—14 | 5.9 | 6.4 | 7.2 |
| G15 and over | 27.2 | 16.7 | 8.7 |

It can be seen that the products of Examples 1, 2 and 3 had the following compositions:-

|  | Ex. 1. | Ex. 2. | Ex. 3. |
|---|---|---|---|
| Glucose | 1.4 | 2.6 | 4.0 |
| D.P. greater than 12 | 33.1 | 23.1 | 15.9 |
| D.P. 2—7 | 60.1 | 67.5 | 71.8 |

Example 4

Three further runs using the conditions of Example 1 were carried out to test the consistency of the results obtained. The composition of the polymer mixture obtained in each of the three runs is shown in Table 3 below. It is clear from this table that the process gives remarkably consistent results.

TABLE 3

| | Run 1 | Run 2 | Run 3 |
|---|---|---|---|
| G1 | 1.5 | 1.5 | 1.4 |
| G2 | 6.6 | 6.8 | 6.8 |
| G3 | 10.8 | 11.4 | 11.3 |
| G4 | 6.6 | 6.9 | 6.9 |
| G5 | 8.2 | 8.1 | 8.1 |
| G6 | 15.9 | 16.7 | 16.8 |
| G7 | 10.4 | 10.4 | 10.0 |
| G8 | 2.5 | 1.7 | 1.7 |
| G9 | 1.3 | 0.9 | 0.8 |
| G10 | 1.0 | 0.9 | 0.8 |
| G11 | 1.0 | 0.9 | 0.8 |
| G12 | 0.9 | 0.8 | 0.8 |
| G13—14 | 6.4 | 6.0 | 5.9 |
| G15 and over | 26.9 | 26.9 | 27.4 |

Example 5

A glucose polymer mixture was prepared by a process similar to that of Example 1 except that step (iii) was effected using 0.1% of the "Ban" enzyme. The composition of the polymer mixture obtained was as follows:-

| | |
|---|---|
| G1 | 1.1 |
| G2 | 5.5 |
| G3 | 9.6 |
| G4 | 6.2 |
| G5 | 6.8 |
| G6 | 15.6 |
| G7 | 11.8 |
| G8 | 2.9 |
| G9 | 1.3 |
| G10 | 1.0 |
| G11 | 0.8 |
| G12 | 0.9 |
| G13—14 | 5.5 |
| G15 and over | 31.2 |

It will be seen that the mixture comprised 1.1% of glucose, 36.7% of polymers of D.P. greater than 12 and 55.5% of polymers of D.P. from 2 to 7.

The mixture was used to replace dextrose in a conventional peritoneal dialysis solution and the resulting solution was clinically tested. The tests were carried out on solutions containing respectively 15%, 10% and 5% of the glucose polymer mixture on a weight to volume basis. The 15% solution withdrew water from the blood of a patient at such a rapid rate (about one litre in half an hour) as to cause discomfort in the patient, and the test was discontinued. Such a rapid withdrawal of water is in any case undesirable in the treatment of a patient suffering from chronic renal disease, when dialysis needs to be effected continuously and the rate of withdrawal of water from the blood plasma should ideally be such as to maintain the water content of the plasma at a level comparable with that of a person having normal kidney function. The 10% solution performed more satisfactorily although here again the clearance of water was somewhat more rapid than would usually be appropriate for treatment of chronic renal failure. The 5% solution was more nearly suitable to effect withdrawal of water at the rate of about 1.0 ml per minute which was deemed to be appropriate for the patient concerned, although it seemed likely that a concentration of lower than 5% might actually be feasible and even preferable.

All of the solutions achieved clearances of water, urea, creatinine, uric acid and phosphates as good or better than could be achieved by a similar solution containing 5% of dextrose instead of the glucose polymer mixture. Surprisingly, it was observed that the clearances of urea, creatinine, uric acid and phosphates were almost as good with the 5% solution as with the 10% solution. This indicates that in general a solution containing 5% or less of the glucose polymer mixture would be preferable for use in continuous ambulatory peritoneal dialysis (where treatment is on a 24 hours per day, seven days per week basis) whereas the 10% and 15% solutions would be more appropriate for intermittent treatment where a more rapid rate of withdrawal of water is tolerable or even desirable.

## Claims

1. The use in the preparation of a peritoneal dialysis composition of an osmotic agent comprising a glucose polymer mixture, said mixture being derived from the hydrolysis of starch and including at least 20% by weight of glucose polymers of D.P. greater than 12.

2. The use of Claim 1 wherein said mixture includes from 20 to 50% by weight of glucose polymers of D.P. greater than 12.

3. The use of Claim 1 wherein said mixture includes less than 3% by weight of glucose.

4. The use of any of Claims 1 to 3 wherein said composition is in the form of an aqueous peritoneal dialysis solution containing from 2 to 15% w/v of said glucose polymer mixture.

5. A process for the preparation of a sterile aqueous solution of a glucose polymer mixture, the mixture containing less than 5% by weight of glucose and more than 15% by weight of glucose polymers having a degree of polymerisation of more than 12, which comprises

(i) gelatinising clean pre-washed starch containing not more than 5% of 1,6-linkages in aqueous suspension with from 0.05 to 0.13%, based on the weight of the suspension, of at least one thermophilic amylase at a pH of from 6.0 to 6.8 and a temperature of 95° to 100°C,

(ii) adjusting the pH of the aqueous mixture from step (i) to 4.5 to 5.0 by the addition of a physiologically acceptable organic acid and increasing the concentration of thermophilic amylase to from 0.15 to 0.25%, on the same basis, by the addition of further amylase and effecting dextrinisation of the gelatinised starch at a temperature of 90° to 95°C so as to obtain a hydrolysis product predominantly consisting of glucose polymers having a degree of polymerisation of more than 12 and containing less than 1% by weight of glucose and less than 2% by weight of each of maltose and maltotriose,

(iii) adjusting the pH of the aqueous mixture from step (ii), if required, to 3.5 to 4.5 by the addition of a physiologically acceptable organic acid and effecting hydrolysis of the dextrinised starch at a temperature of 65° to 75°C by means of a bacterial alpha-amylase which is capable, under these conditions, of producing less than 5% of glucose, based on the weight of the dextrinised starch, and continuing hydrolysis under these conditions until a glucose polymer mixture of the desired composition is obtained, and

(i) adjusting the pH of the aqueous mixture from step (iii) to 2.0 to 3.0 by the addition of a physiologically

EP 0 115 911 B2

acceptable organic acid and heating the mixture at a temperature of 96° to 100°C so as to inactivate the enzymes.

6. The process of Claim 5, wherein the amount of bacterial alpha-amylase used in step (iii) is from 0.1 to 3.0% based on the total weight of the aqueous mixture.

**Patentansprüche**

1. Verwendung eines osmotischen Mittels aus einer Glukosepolymermischung, wobei die Mischung auf die Hydrolyse von Stärke zurückgeht und wenigstens 20 Gew.-% Glukosepolymere mit einem Polymerisationsgrad von mehr als 12 umfaßt, zur Herstellung einer peritonealen Dialysezubereitung.

2. Verwendung nach Anspruch 1, wobei die Mischung 20 bis 50 Gew.-% Glukosepolymere mit einem Polymerisationsgrad von mehr als 12 umfaßt.

3. Verwendung nach Anspruch 1, wobei die Mischung weniger als 3 Gew.-% Glukose umfaßt.

4. Verwendung nach einem der Ansprüche 1 bis 3, wobei die Zubereitung in Form einer wäßrigen peritonealen Dialyselösung vorliegt, die 2 bis 15% (Gew./Vol.) der Glukosepolymermischung enthält.

5. Verfahren zur Herstellung einer sterilen wäßrigen Lösung einer Glukosepolymermischung, wobei die Mischung weniger als 5 Gew.-% Glukose und mehr als 15 Gew.% Glukosepolymerer mit einem Polymerisationsgrad von mehr als 12 enthält, welches darin besteht
(i) saubere vorgewaschene Stärke, welche nicht mehr als 5% 1,6-Verknüpfungen enthält, in einer wäßrigen Suspension mit 0,05 bis 0,13%, bezogen auf das Gewicht der Suspension, wenigstens einer thermophilen Amylase bei einem pH von 6,0 bis 6,8 sowie bei einer Temperatur von 95 bis 100°C zu gelatinisieren,
(ii) den pH der wäßrigen Mischung aus der Stufe (i) auf 4,5 bis 5,0 durch Zugabe einer physiologisch verträglichen organischen Säure einzustellen und die Konzentration der thermophilen Amylase von 0,15 auf 0,25%, bezogen auf die gleiche Basis, durch Zugabe einer weiteren Amylase zu erhöhen und die Dextrinisierung der gelatinisierten Stärke bei einer Temperatur von 90° bis 95°C zur Gewinnung eines Hydrolyseprodukts zu bewirken, das überwiegend aus Glukosepolymeren mit einem Polymerisationsgrad von mehr als 12 besteht und weniger als 1 Gew.-% Glukose und weniger als 2 Gew.-% jeweils Maltose und Maltotriose enthält,
(iii) den pH der wäßrigen Mischung aus der Stufe (ii) erforderlichenfalls auf 3,5 bis 4,5 durch die Zugabe einer physiologisch verträglichen organischen Saure einzustellen und die Hydrolyse der dextrinisierten Stärke bei einer Temperatur von 65° bis 75°C mittels einer bakteriellen alpha-Amylase zu bewirken, die unter diesen Bedingungen dazu in der Lage ist, weniger als 5% Glukose, bezogen auf das Gewicht der dextrinisierten Stärke, zu erzeugen und die Hydrolyse unter diesen Bedingungen fortzusetzen, bis eine Glukosepolymermischung mit der gewünschten Zusammensetzung erhalten worden ist, und
(iv) den pH der wäßrigen Mischung aus der Stufe (iii) auf 2,0 bis 3,0 durch die Zugabe einer physiologisch verträglichen organischen Säure einzustellen und die Mischung auf eine Temperatur von 96° bis 100°C zur Inaktivierung der Enzyme zu erhitzen.

6. Verfahren nach Anspruch 5, wobei die Menge der bakteriellen alpha-Amylase, die in der Stufe (iii) eingesetzt wird, 0,1 bis 3,0%, bezogen auf das Gesamtgewicht der wäßrigen Mischung, beträgt.

**Revendications**

1. Utilisation, dans la préparation d'une composition pour dialyse péritonéale, d'un agent osmotique comprenant un mélange de polymères de glucose, ledit mélange étant dérivé de l'hydrolyse d'amidon et comprenant au moins 20% en poids de polymères de glucose de degré de polymérisation supérieur à 12.

2. Utilisation suivant la revendication 1, dans laquelle ledit mélange contient 20 à 50% en poids de polymères de glucose de degré de polymérisation supérieur à 12.

3. Utilisation suivant la revendication 1, dans laquelle ledit mélange contient moins de 3% en poids de glu-

9

cose.

4. Utilisation suivant l'une quelconque des revendications 1 à 3, dans laquelle ladite composition est sous la forme d'une solution aqueuse pour dialyse péritonéale contenant 2 à 15% en poids/volume dudit mélange de polymères de glucose.

5. Procédé de préparation d'une solution aqueuse stérile d'un mélange de polymères de glucose, le mélange contenant moins de 5% en poids de glucose et plus de 15% en poids de polymères de glucose ayant un degré de polymérisation supérieur à 12, qui consiste

(i) à gélatiniser de l'amidon propre, préablement lavé, ne contenant pas plus de 5% de liaisons 1,6 en suspension aqueuse avec 0,5 à 0,13%, sur la base du poids de la suspension, d'au moins une amylase thermophile à un pH de 6,0 à 6,8 et à une température de 95° à 100°C,

(ii) à ajuster le pH du mélange aqueux venant de l'étape (i) à une valeur de 4,5 à 5,0 par l'addition d'une acide organique physiologiquement acceptable et à élever la concentration en amylase thermophile de 0,15 à 0,25%, sur la même base, par l'addition d'une quantité supplémentaire d'amylase et à effectuer la dextrinification de l'amidon gélatilisé à une température de 90 à 95°C de manière à obtenir un produit d'hydrolyse principalement constitué de polymères de glucose ayant un degré de polymérisation supérieur à 12 et contenant moins de 1% en poids de glucose et moins de 2% en poids de maltose ainsi que de maltotriose,

(iii) à ajuster le pH du mélange aqueux venant de l'étape (ii), le cas échéant, à une valeur de 3,5 à 4,5 par l'addition d'une acide organique physiologiquement acceptable et à effectuer l'hydrolyse de l'amidon dextrinifié à une température de 65° à 75°C au moyen d'une alpha-amylase bactérienne qui est capable, dans ces conditions, de produire moins de 5% de glucose, sur la base du poids de l'amidon dextrinifié, et à poursuivre l'hydrolyse dan ces conditions jusqu'à ce qu'un mélange de polymères de glucose de la composition désirée ait été obtenu, et

(iv) à ajuster le pH dy mélange aqueux venant de l'étape (iii) à une valeur de 2,0 à 3,0 par l'addition d'un acide organique physiologiquement acceptable et à chauffer le mélange à une température de 96 à 100°C de manière à désactiver les enzymes.

6. Procédé suivant la revendication 5, dans laquel la quantité d'alpha-amylase bactérienne utilisée dans l'étape (iii) va de 0,1 à 3,0% sur la base du poids total du mélange aqueux.